Europäisches Patentamt

**European Patent Office**

**Office européen des brevets**

(11) Numéro de publication: **0 227 522**
**A1**

## (12) DEMANDE DE BREVET EUROPEEN

(21) Numéro de dépôt: **86402552.3**

(22) Date de dépôt: **18.11.86**

(51) Int. Cl.⁴: **A 61 B 6/04**

(30) Priorité: **26.11.85 FR 8517478**

(43) Date de publication de la demande:
**01.07.87 Bulletin 87/27**

(84) Etats contractants désignés: **DE IT NL**

(71) Demandeur: **THOMSON-CGR**
**13, square Max-Hymans**
**F-75015 Paris (FR)**

(72) Inventeur: **Jarin, Jean-Pierre**
**THOMSON-CSF SCPI 19, avenue de Messine**
**F-75008 Paris (FR)**

(74) Mandataire: **Grynwald, Albert et al**
**THOMSON-CSF SCPI 19, avenue de Messine**
**F-75008 Paris (FR)**

(54) **Appareil à éléments mobiles par glissière du type à roulement.**

(57) Des moyens destinés à la protection de glissières (7.8) à roulement servent à assurer un mouvement relatif entre deux éléments (3.4) tels que constitués par un socle et support de panneau (2), dans une table d'examen (1). Les moyens de protection comportent une bande souple (30) formant écran devant des chemins de roulement (13) à protéger. que comportent les glissières (7.8).

FIG_1

**Description**

APPAREIL A ELEMENTS MOBILES PAR GLISSIERE DU TYPE A ROULEMENT

La présente invention se rapporte à un appareil dans lequel deux éléments sont rendus mobiles l'un par rapport à l'autre, grâce à des glissières du type à roulement. L'invention concerne plus précisément des moyens pour la protection de telles glissières.

Des glissières à roulement sont utilisées dans des appareils de types variés, pour assurer un déplacement relatif entre deux éléments. particulièrement quand ce déplacement doit présenter certaines qualités telles que par exemple. précision et souplesse, et doit s'effectuer sous une faible force d'actionnement.

Quel que soit le domaine auquel appartiennent ces appareils, la qualité du déplacement n'est conservée qu'en protégeant la glissière contre des agents extérieurs qui risquent de dégrader les qualités du roulement. Un exemple en est donné avec les tables d'examen radiologique, où il est courant d'utiliser de telles glissières et d'en prévoir la protection.

Ce type de glissière comporte deux pièces munies chacune selon leur longueur, d'un chemin de roulement. Chaque pièce est destinée à être fixée à l'un des éléments entre lesquels on veut obtenir un déplacement : par exemple d'une part un élément supportant un panneau porte-patient, et d'autre part un autre élément formant le socle de la table d'examen. La glissière comporte en outre un dispositif de roulement, formée par des moyens de roulement tels par exemple des billes ou des galets. maintenus dans une cage. Le dispositif de roulement est engagé dans les deux chemins de roulement. dans la longueur desquels il est déplacé avec le déplacement des éléments mobiles.

En général des moyens de protection sont disposés en vue de protéger les chemins de roulement. sur des parties de ces derniers qui se trouvent dégagées par suite d'un déplacement des éléments, et qui sont alors particulièrement vulnérables ; ces parties dégagées se trouvant du côté d'une extrémité ou de l'autre de ces chemins de roulement. suivant le sens du déplacement des éléments.

Dans l'art antérieur, les moyens de protection de glissières consistent en au moins un capot. fixé sur le côté d'un des éléments mobiles, et dont la longueur doit être au moins égale à la course maximum du déplacement, pour protéger une partie dégagée d'un chemin de roulement ; un capot semblable devant être placé de l'autre côté de l'élément mobile, pour protéger l'extrémité opposé du chemin de roulement. et assurer la protection de ce dernier quelque soit le sens du déplacement.

Un des inconvénients de cette solution réside dans son encombrement. cet encombrement étant encore augmenté du fait que la longueur de cette protection est égale à deux fois la course. Il est à remarquer que cet encombrement est particulièrement gênant dans le cas des tables d'examen. où un accès aisé au patient est nécessaire.

Pour tenter de diminuer cet encombrement. ces capots sont parfois réalisés selon des systèmes télescopiques ou à soufflets, de manière à présenter des longueurs réduites quand l'élément mobile n'a pas été déplacé selon sa course maximum. Ceci tend à diminuer l'encombrement qui pourtant reste non négligeable. mais accroît la complexité de l'installation. et conduit ainsi à une augmentation du coût tant en ce qui concerne le prix et le montage des pièces qu'en ce qui concerne l'entretien.

La présente invention concerne un appareil dans lequel deux éléments sont rendus mobiles l'un par rapport à l'autre grâce à au moins une glissière du type à roulement, et dans lequel des moyens de protection des glissières sont agencés d'une manière nouvelle qui leur permet, d'une part de présenter un encombrement négligeable pratiquement compris dans le volume de glissière, et leur permet d'autre part d'être constitués par des moyens simples, de mise en oeuvre aisée. qui conduisent à un moindre coût et une grande souplesse de manoeuvre.

Selon l'invention. un appareil à éléments mobiles par glissières du type à roulement, chaque glissière comportant une première et une seconde pièces munies chacune d'un chemin de roulement, lesdites première et seconde pièces étant fixées respectivement à un premier et à un second élément mobiles l'un par rapport à l'autre. un dispositif de roulement étant engagé dans chacun desdits chemins de roulement et se déplaçant dans la longueur de ces derniers, ledit appareil comportant en outre des moyens de protection destinés à protéger au moins un desdits chemins de roulement, est caractérisé en ce que lesdits moyens de protection comportent une bande souple disposée devant ledit chemin de roulement, ladite bande souple formant une boucle de manière à entourer ledit chemin de roulement selon sa longueur. afin d'assurer la protection dudit chemin de roulement quelles que soient les positions respectives desdits premier et second éléments.

L'invention sera mieux comprise grâce à la description qui suit. faite à titre d'exemple non limitatif. et aux trois figures annexées parmi lesquelles :

    - la figure 1 est une vue en perspective qui montre la configuration générale d'un appareil selon l'invention comportant deux éléments mobiles l'un par rapport à l'autre grâce à des glissières ;

    -la figure 2 représente des glissières et des moyens de protection déjà montrés à la figure 1. et vus selon une direction représentée par une flèche A à la figure 1 ;

    -la figure 3 représente les deux éléments et les glissières déjà montrées à la figure 1 et vus selon une seconde direction représentée par une flèche B à la figure 1.

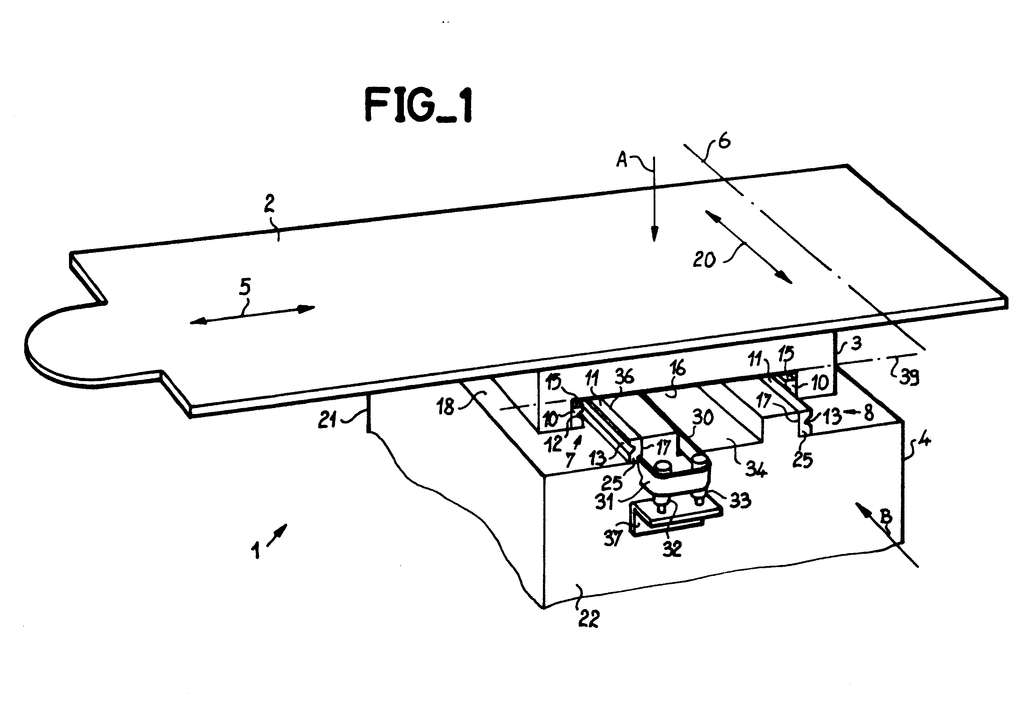

La figure 1 montre par une vue en perspective. la configuration générale d'un appareil à éléments mobiles par glissières conformes à l'invention. Dans l'exemple non limitatif de la description, cet appareil est représenté par une table d'examen 1 radiologique.

La table d'examen 1 comporte un panneau 2 porte-patient, supporté par un premier élément 3. Le premier élément 3 est lui-même supporté par un second élément 4 constituant par exemple un socle dont la base (non représentée) repose sur le sol.

Le panneau 2 peut être déplacé selon sa longueur comme montré par la flèche 5, grâce à des moyesn conventionnels, non représentés, disposés par exemple entre le panneau 2 et le premier élément 3.

Afin de permettre également un déplacement du panneau 2 selon un axe 6 transversal à sa longueur, le premier élément 3 et rendu mobile par rapport au second élément 4, parallèlement à l'axe transversal 6, grâce à une première et à une seconde glissière 7, 8.

Les glissières 7,8 sont d'un type couramment utilisé, comportant des moyens de roulement tels que des billes ou des galets par exemple (non représentés), et sont montées d'une manière en elle-même connue. Chaque glissière 7,8 comporte une première et une seconde pièce 10,11 qui sont solidarisées respectivement au premier et au second éléments 3,4, parallèlement à l'axe transversal 6. Les première et seconde pièces comportent chacune un chemin de roulement, respectivement 12,13 ; les première et seconde pièces 10,11 étant fixées de manière que les chemins de roulement 12,13 soient orientés l'un vers l'autre, sensiblement en vis à vis. Chaque glissière 7,8 comporte en outre un dispositif de roulement (non représenté sur la figure 1), constitués par des billes ou galets maintenus dans une cage : ce dispositif de roulement étant engagé dans les deux chemins de roulement d'une même glissière 7,8 et se déplaçant dans la longueur des chemins de roulement quand les premier et second éléments 3,4 sont déplacés l'un par rapport à l'autre.

Dans l'exemple non limitatif décrit, les premières pièces 10 sont fixées contre des bords 15 d'un décrochement 16 aménagé dans la partie inférieure du premier élément 3 : les premières pièces 10 sont ainsi pratiquement à l'intérieur du premier élément 3, de sorte que les chemins de roulement 12 qu'elles comportent sont protégés contre les salissures.

Les secondes pièces 11 sont fixées en appui contre des épaulements 17 que comporte le second élément 4 sur sa partie supérieure 18 ; les secondes pièces 11 étant ainsi en saillie par rapport à cette partie supérieure 18, ainsi que les seconds chemins de roulement 13 qu'elles comportent.

Aussi, selon que le premier élément 3 est déplacé selon la seconde flèche 20 vers un premier côté 21 ou un second côté 22 du second élément 4, les chemins de roulement 13 portés par les secondes pièces 11 se trouvent dégagées sur des tronçons situés, soit du côté de leur première extrémité 25 ainsi que dans l'exemple montré sur la figure 1, soit du côté de l'extrémité opposée (non visible sur la figure 1). Ces tronçons seraient exposés à être salis, en l'absence des moyens de protection décrits ci-après.

Selon une caractéristique de l'invention, la protection des seconds chemins de roulement 13 est assurée grâce à une bande souple 30 dont le plan 31 est destiné à former écran devant un chemin de roulement. Pour plus de clarté de la figure 1, la bande 30 n'est que partiellement représentée, et seulement pour la protection du second chemin de roulement 13 porté par la seconde pièce 11 de la première glissières 7 ; mais il doit être entendu qu'une même protection peut être réalisée pour la seconde glissière 8, et aussi si cela était nécessaire pour les premiers chemins de roulement 12 portés par les premières pièces 10, dans le cas par exemple où ceux-ci ne seraient pas montés selon une configuration dans laquelle ils sont protégés par le premier élément 3.

Dans l'esprit de l'invention la bande souple 30 est formée selon une boucle qui entoure le chemin de roulement 13 selon sa longueur ; la bande souple 30 forme une boucle qui passe à l'arrière du chemin de roulement 13 et qui, devant ce dernier, est refermée sur elle-même par le dispositif de roulement précédemment mentionné.

La bande souple peut être réalisée par exemple en caoutchouc, ou en tout autre matériau lui permettant de comporter, d'une part, la souplesse nécessaire pour tourner autour de moyens de renvoi tels que par exemple des premier et second rouleaux 32,33, et lui permettant d'autre part, de comporter une robustesse mécanique suffisante à éviter qu'elle ne se détende trop ou de manière non uniforme. La bande souple 30 doit en outre présenter une résistance suffisante aux attaques chimiques des différents produits qui peuvent l'atteindre, soit en cours d'examen d'un patient, soit pour l'entretien de la table d'examen 1.

La bande souple 30 est disposée le long du chemin de roulement 13, sur des parties non occupées par le dispositif de roulement, son plan 31 étant sensiblement parallèle au plan moyen du chemin de roulement 13, soit sensiblement vertical dans l'exemple non limitatif représenté à la figure 1.

La bande souple 30 étant solidarisée au dispositif de roulement (non visible sur la figure 1), elle est tendue le long du chemin de roulement 13 dont elle dépasse la première extrémité 25 avant de tourner une première fois vers le dos 36 du moyen de roulement 13, grâce à un premier moyen de renvoi formé par le premier rouleau 32. La bande souple 30 tourne ensuite une seconde fois autour d'un second moyen de renvoi 33 pour longer le dos 36 du chemin de roulement 13, en passant dans un espace 34 aménagé entre les épaulements 17 auxquels sont fixées les secondes pièces 11 ; le second moyen de renvoi 33 étant formé par un second rouleau porté comme le premier rouleau par une équerre 37 fixée sur le côté 22 du second élément 4. La bande souple 30 rejoint ensuite le premier côté 21 de l'élément 4, où elle réalise un trajet semblable à celui qui vient d'être expliqué pour rejoindre le dispositif de roulement.

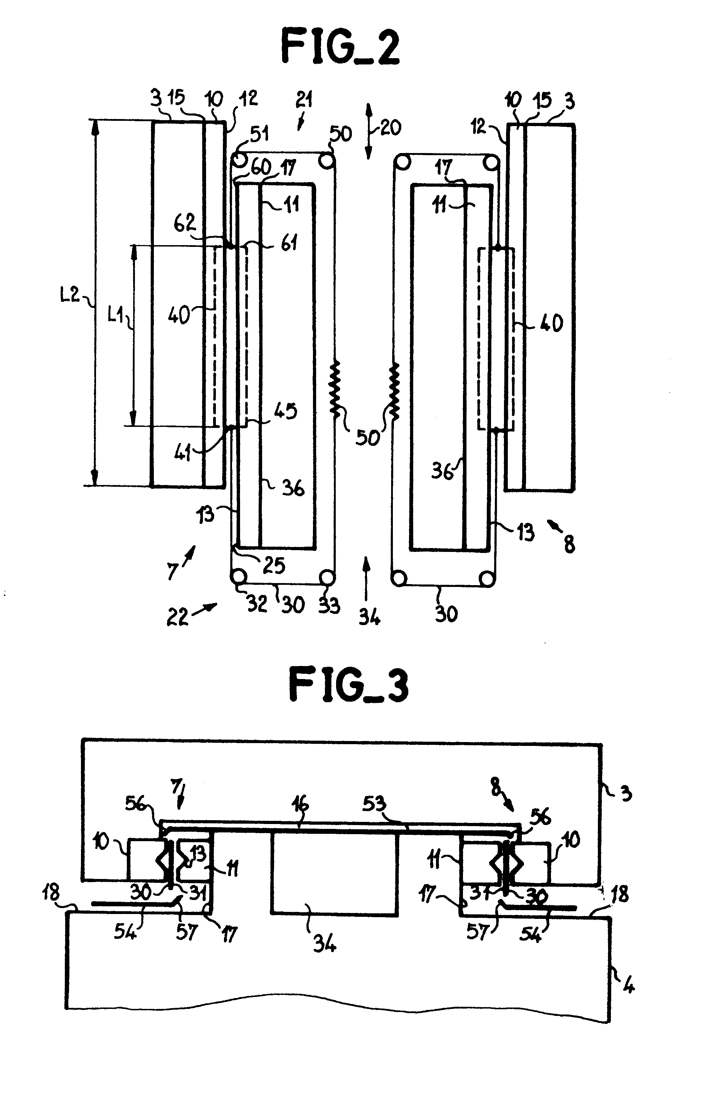

La boucle formée par la bande souple 30 apparaît plus clairement, de manière schématique, sur la figure 2 qui montre les première et seconde pièces 10,11 des glissières 7,8 par une vue de dessus selon une flèche A représentée à la figure 1, et telles que ces glissières apparaîtraient si le premier élément 3 était coupé suivant un plan parallèle au panneau 2 et suivant une ligne en traits pointillés 39 montrée à la figure 1.

La figure 2 montre de manière schématique les première et seconde glissières 7,8. Les premières pièces 10 sont fixées contre les bords 15 du premier élément 3, les secondes pièces 11 étant fixées en appui contre les épaulements 17 du second élément 4, comme il a été précédemment décrit.

Chaque glissière 7,8 comporte un dispositif de roulement 40, précédemment mentionné, engagé à la fois dans le premier et le second chemin de roulement 12,13. Le dispositif de roulement 40 comporte une première longueur L1 inférieure à une seconde longueur L2 des chemins de roulement 12,13 ; le dispositif de roulement 40 étant déplacé à l'intérieur de cette seconde longueur L2 dans un même sens que le déplacement du premier élément 3 par rapport au second élément 4. Pour simplifier la description, l'explication qui suit est donnée pour la première glissière 7, mais constitue un exemple valable également pour la seconde glissière 8.

Une première extrémité 41 de la bande souple 30 est fixée de manière conventionnelle à une première extrémité 45 du dispositif de roulement 40, à partir de laquelle la bande souple 30 est tendue entre les deux chemins de roulement 12,13 jusqu'à dépasser la première extrémité 25 du second chemin de roulement 13, après lequel elle tourne autour du premier rouleau 32 en direction du dos 36 de la seconde pièce 11 ; la bande souple 30 tourne ensuite autour du second rouleau 33 et passe dans l'espace 34 aménagé entre les deux épaulements 17, pour longer le dos 30 de la seconde pièce 11. Dans l'exemple non limitatif décrit, la bande souple 30 a été séparée en deux parties reliées par un dispositif de tension 50, constitué par un ressort par exemple. La bande souple 30 longe le dos 36 jusqu'à rejoindre le premier côté 21 du second élément 4, où elle tourne autour d'un troisième et d'un quatrième rouleaux 51, 52 pour longer à nouveau le second chemin de roulement 13 du côté de la seconde extrémité 60 de ce dernier. La seconde extrémité 62 de la bande souple 30 est elle-même fixée à la seconde extrémité 61 du dispositif de roulement 40.

Dans ces conditions la bande souple 30 constitue une boucle, fermée sur elle-même par l'intermédiaire du dispositif de roulement 40 devant le second chemin de roulement 13.

La bande souple 30 est mise en mouvement sur elle-même quand le dispositif de roulement 40 est déplacé dans le même sens que ce dernier. Le second chemin de roulement 13 étant ainsi entouré selon sa longueur l1 par la bande souple 30, les tronçons de chemin de roulement susceptibles d'être dégagés par le déplacement du premier élément 3 reste toujours protégé par la bande souple 30.

Le dispositif de tension 50 permet d'ajuster et de maintenir la tension de la bande souple 30, nécessaire à son déplacement correct, tout en évitant que ce déplacement n'exige une force supplémentaire d'actionnement trop importante : un résultat semblable pouvant être obtenu également d'une autre manière, en ajustant la position de l'un des rouleaux 32, 33 par exemple, tout en conservant aux moyens de protection un encombrement considérablement réduit par rapport à l'art antérieur.

Les moyens de protection comportent, outre la bande souple 30, des capots 53, 54 montrés à la figure 3, qui permettent de parfaire la protection contre des ruissellements de liquide.

La figure 3 montre le premier et le second éléments 3,4 vue selon une flèche B montrée à la figure 1, c'est-à-dire selon le second côté 22 du second élément 4, de manière à représenter la position relative des différents éléments des moyens de protection ; la partie de bande souple 30 parallèle au côté 22 ainsi que les rouleaux 32, 33 ne sont pas représentés pour plus de clarté de la figure.

Un capot supérieur 53 est disposé au-dessus de chaque glissière 7,8, ce capot supérieur étant, dans l'exemple non limitatif décrit, commun ou non aux deux glissières 7,8. Le capot supérieur 53 est disposé dans la partie inférieure du premier élément 3, entre le décrochement 16 dont est muni ce dernier et le plan supérieur 18 du second élément 4 auquel il est fixé par exemple. Le capot supérieur 53 comporte des bords extérieurs 56, parallèles aux glissières 7,8, qui débordent par rapport aux bandes souples 30, et qui sont courbés vers le second élément 4, de manière à éviter que des liquides ne ruissellent entre les chemins de roulement 13, et la bande souple 30.

Un second capot 54 est disposé du côté de chaque glissière 7,8. Chaque second capot 54 est formé selon une plaque placée à un niveau compris entre le plan supérieur 18 du second élément 4 et la bande souple 30. Chaque second capot 54 comporte un bord intérieur 57 orienté vers un épaulement 17, et parallèle à la glissière 7,8, ce bord intérieur 57 étant engagé entre le bas de la bande souple 30 et le plan supérieur 18 du second élément 4. Le bord intérieur 57 est courbé en direction du capot supérieur 53, de manière à constituer une gouttière permettant d'évacuer à distance des liquides répandus sur le plan 31 de la bande souple 30.

Cette description constitue un exemple non limitatif, permettant de montrer comment peut être assurée la protection de chemins de roulement de glissières, avec des moyens simples et sous un faible encombrement, grâce notamment à une bande souple 30 entourant un chemin de roulement 13 selon sa longueur L1.

L'invention est applicable à la protection de chemins de roulement, dans tout appareil dans lequel un premier élément est déplacé par rapport à un second élément grâce à une ou des glissières du type à roulement.

## Revendications

1. Appareil à éléments mobiles par glissières du type à roulement, chaque glissière (7,8) comportant une première et une seconde pièces (10.11) munies chacune selon leur longueur (L1) d'un chemin de roulement (12.13). lesdites première et seconde pièces (10.11) étant fixées respectivement à un premier et à un second élément (3.4) mobiles l'un par rapport à l'autre, les chemins de roulement (12 . 13) étant orientés l'un vers l'autre, un dispositif de roulement (40) étant engagé à la fois dans chacun desdits chemins de roulement (12.13) et se déplaçant à l'intérieur de la longueur (L1) de ces derniers. ledit appareil (1) comportant en outre des moyens de protection (30-54) destinés à protéger au moins un desdits chemins de roulement (12.13). caractérisé en ce que lesdits moyens de protection (30-54) comportent une bande souple (30) disposée devant ledit chemin de roulement à protéger (13), ladite bande souple formant une boucle fermée sur elle-même par l'intermédiaire dudit dispositif de roulement (40) de manière à entourer ledit chemin de roulement (13) selon sa longueur (L1). afin d'assurer la protection dudit chemin de roulement (13) quelles que soient les positions respectives desdits premier et second éléments (3,4).

2. Appareil selon la revendication précédente, caractérisé en ce que ladite bande souple (30) est fixée par ses extrémités (41,62) aux extrémités (45,61) dudit dispositif de roulement (40), et est entraînée dans un déplacement sur elle-même par le déplacement dudit dispositif de roulement (40).

3. Appareil selon la revendication précédente, caractérisé en ce que ladite bande souple (30) s'étend à partir dudit dispositif de roulement (40). vers les extrémités (25,60) dudit chemin de roulement (13) jusqu'à les dépasser avant de tourner autour de moyens de renvoi (32.33.51). afin de constituer une boucle en longeant l'arrière (36) de ladite seconde pièce (11).

4. Appareil selon la revendication 3. caractérisé en ce que lesdits moyens de protection (30) comportent en outre un dispositif de tension (50).

5. Appareil selon la revendication 4. caractérisé en ce que ladite bande souple (30) est séparée en deux parties qui sont reliées à l'arrière (36) de ladite pièce (11) par ledit dispositif de tension (50).

6. Appareil selon la revendication 5. caractérisé en ce que ledit dispositif de tension (50) est un ressort.

7. Appareil selon l'une des revendications précédentes. caractérisé en ce que lesdits moyens de protection (30-54) comportent en outre au moins un capot supérieur (53) disposé au-dessus dudit chemin de roulement (13) et de ladite bande souple (30). ledit capot supérieur comportant un bord extérieur (56) débordant par rapport au plan (31) de ladite bande souple (30). ledit bord extérieur (54) étant courbée vers le second élément (4), afin d'éviter le ruissellement de liquide entre ledit chemin de roulement (13) et ladite bande souple (30).

8. Appareil selon l'une des revendications précédentes. caractérisé en ce que les moyens de protection (30-53) comportent en outre un second capot (54) disposé sous ladite bande souple (30). ledit second capot comportant un bord intérieur (57) dépassant le plan de ladite bande souple (30) vers ledit chemin de roulement (13). ledit bord intérieur étant relevé vers ledit premier capot (53) de manière à constituer une gouttière pour l'évacuation à distance de liquides.

# FIG_1

# FIG_2

# FIG_3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | US-A-3 967 126  (G.W. OTTO Jr.) <br> * Colonne 2, ligne 28 - colonne 3, ligne 44; figures 1,3,4 * | 1 | A 61 B    6/04 |
| A |  | 7 |  |
|  | --- |  |  |
| Y | DE-A-2 840 520  (SIEMENS AG) <br> * Page 4, lignes 24-32;  page 6, ligne 15 - page 7, ligne 10; figures 1,2 * | 1 |  |
| A |  | 2,3 |  |
|  | --- |  |  |
| A | DE-B-1 161 382  (KOCH ET STERZEL KOMMANDITGESELLSCHAFT) <br> * Colonne 1, lignes 1-6; colonne 3, lignes 13-19,27-31; figures 1,2 * | 1 |  |
|  |  |  | **DOMAINES TECHNIQUES RECHERCHES (Int. Cl.4)** |
|  | --- |  | A 61 B <br> E 01 B |
| A | US-A-2 736 931  (N. LEVINE) <br> * Colonne 1, ligne 18 - colonne 2, ligne 18; figures 1-4 * | 1-3 |  |
|  | --- |  |  |
| A | DE-B-1 766 449  (KOCH & STERZEL) <br> * Colonne 1, lignes 37-48; colonne 2, lignes 17-22; colonne 3, lignes 3-9; figure 2 * | 4-6 |  |
|  | ---                  -/- |  |  |

Le présent rapport de recherche a été établi pour toutes les revendications

| Lieu de la recherche | Date d'achèvement de la recherche | Examinateur |
|---|---|---|
| LA HAYE | 29-01-1987 | FERRIGNO, A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82

**Office européen
des brevets**

## RAPPORT DE RECHERCHE EUROPEENNE

| | **DOCUMENTS CONSIDERES COMME PERTINENTS** | | Page 2 |
|---|---|---|---|
| **Catégorie** | Citation du document avec indication, en cas de besoin, des parties pertinentes | **Revendication concernée** | **CLASSEMENT DE LA DEMANDE (Int. Cl.4)** |
| A | US-A-2 700 735 (A.J. KIZAUR) <br> * Colonne 1, lignes 15-26; colonne 2, lignes 48-74; colonne 4, lignes 46-59; figures 6,7 * <br><br> ----- | 8,9 | |
| | | | **DOMAINES TECHNIQUES RECHERCHES (Int. Cl.4)** |
| | Le présent rapport de recherche a été établi pour toutes les revendications | | |

| Lieu de la recherche | Date d'achèvement de la recherche | Examinateur |
|---|---|---|
| LA HAYE | 29-01-1987 | FERRIGNO, A. |

**CATEGORIE DES DOCUMENTS CITES**

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

& : membre de la même famille, document correspondant

OEB Form 1503 03 82